# EUROPEAN PATENT APPLICATION

(11) **EP 4 527 445 A1**
(43) Date of publication of application: **26.03.2025**
(21) Application number: 24201917.2
(22) Date of filing: 23.09.2024
(51) Int. Cl.: A61N 1/36

(54) **ELECTRICAL STIMULATION DEVICE**

(30) Priority: 25.09.2023 TW 112136944; 29.05.2024 TW 113119906
(71) Applicant: Gimer Medical. Co., Ltd, New Taipei City 221 (TW)
(72) Inventor: LIN, Wei-Tso, 221 New Taipei City (TW); LU, Kuo-Hsiang, 221 New Taipei City (TW)
(74) Representative: Viering, Jentschura & Partner mbB Patent- und Rechtsanwälte

(57) **Abstract**

An electrical stimulation device (10) includes an electrode assembly (11) and an electrical stimulator (12). The electrical stimulator (12) generates an electrical stimulation signal (ES) and is coupled to the electrode assembly (11). The electrical stimulation signal (ES) is transmitted to a target region (T) of an organism (20) through the electrode assembly (11). The electrical stimulation signal (ES) contains a plurality of burst signals (B), and the burst signals (B) have the burst frequency between 0.1 Hz and 1,000 Hz. Each burst signal (B) contains a plurality of pulses (P), and the pulses (P) have the pulse frequency between 1 kHz (kilohertz) and 1,000 kHz. The electrical stimulation signal (ES) reduces the level of cyclooxygenase-2 (COX-2) of the organism (20).

## Description

### BACKGROUND

### Technical Field

The present invention relates to the technical field of electrical stimulation, particularly to an electrical stimulation device.

### Related Art

Cyclooxygenase (COX) is an important enzyme involved in the inflammatory reaction. When tissues of an organism are stimulated to activate COX, the ω-6 fatty acids on a cell membrane are converted into prostaglandin E2 (PGE2), prostaglandin F2α (PGF2α), thromboxane and other prostaglandins in a large amount, causing red, swelling, heat, pain, vasodilatation and other reactions. COX includes COX-1 and COX-2. Among these, COX-1 is present in various tissues of an organism and is responsible for regulating normal cellular activities, such as protecting the stomach wall and platelets from coagulation, whereas COX-2 is induced only when the body is inflamed.

Analgesics can be classified into three major groups, from weak to strong, according to their analgesic strength, acetaminophen, nonsteroidal anti-inflammatory drugs (NSAIDs) and opioid analgesics. When inflammation occurs in vivo, activated cyclooxygenase converts arachidonic acid into prostaglandins and further produces more inflammatory factors, causing inflammation and pain. NASIDs mainly achieve the effects of diminishing inflammation, relieving pain and/or relieving fever and pain by inhibiting COX and further blocking the process of converting arachidonic acid through COX.

In addition, there are also new analgesics of NSAIDs capable of selectively inhibiting COX-2 without affecting COX-1 at the therapeutic dose. The drugs have the advantage of low incidence of gastrointestinal mucosa injury. However, although the drugs can relieve existing disease characteristics, the accumulation of the drugs may cause problems such as side effects.

### SUMMARY

However, there is currently no electrical stimulation therapy that inhibits cyclooxygenase-2 (COX-2) without the side effects caused by drug accumulation. Accordingly, the present disclosure provides an electrical stimulation device, which can reduce COX-2 without causing nerve injury.

In some embodiments, an electrical stimulation device includes an electrode assembly and an electrical stimulator. The electrical stimulator is coupled to the electrode assembly and generates an electrical stimulation signal. The electrical stimulation signal is transmitted to a target region of an organism through the electrode assembly. The electrical stimulation signal contains a plurality of burst signals, and the burst signals have the burst frequency between 0.1 Hz and 1,000 Hz. Each burst signal contains a plurality of pulses, and the pulses have the pulse frequency between 1 kHz (kilohertz) and 1,000 kHz. The electrical stimulation signal reduces a level of cyclooxygenase-2 of the organism.

In some embodiments, the electrical stimulation signal reduces the level of the cyclooxygenase-2 by inhibiting a protein expression of the cyclooxygenase-2.

In some embodiments, reducing the level of the cyclooxygenase-2 of the organism occurs within 24 hours after receiving the electrical stimulation signal.

In some embodiments, the electrical stimulator generates the electrical stimulation signal for a cumulative time less than or equal to 12 hours per day.

In some embodiments, the electrical stimulation signal further reduces the levels of brain-derived neurotrophic factor (BDNF) and/or substance P of the organism.

In some embodiments, the electrical stimulator uses the electrical stimulation signal to inhibit neuroinflammatory response of the organism.

In some embodiments, the electrical stimulation signal inhibits and/or relieves at least a part of pain on a nerve conduction path of the target region.

In some embodiments, after the organism receives the electrical stimulation signal for less than or equal to 12 hours, the electrical stimulation signal relieves pain of the organism, relieves symptoms of the organism, reduces neural sensitivity of the organism or relieves overactive reaction of the organism and maintains for at least 1 hour.

In some embodiments, the electrical stimulation signal reduces the level of the cyclooxygenase-2 by inhibiting a protein expression of the cyclooxygenase-2.

In some embodiments, reducing the level of the cyclooxygenase-2 of the organism occurs within 24 hours of receiving the electrical stimulation signal at the target region.

In some embodiments, the electrical stimulator generates the electrical stimulation signal for a cumulative time less than or equal to 12 hours per day.

In some embodiments, after the organism receives the electrical stimulation signal for less than or equal to 12 hours, the electrical stimulation signal relieves pain of the organism, relieves symptoms of the organism, reduces neural sensitivity of the organism or relieves overactive reaction of the organism and maintains for at least 1 hour.

In some embodiments, the electrical stimulation signal further inhibits the levels of brain-derived neurotrophic factor and/or substance P of the organism, inhibits neuroinflammatory response of the organism or inhibits and/or relieves at least a part of pain on a nerve conduction path of the target region.

In summary, according to any of the embodiments, the electrical stimulation device has the effect of reducing COX-2 without causing the nerve injury. The electrical stimulation device has the effects of reducing the level of the brain-derived neurotrophic factor, reducing the level of the substance P, inhibiting the neuroinflammatory response, inhibiting and/or relieving pain, relieving symptoms, reducing nerve sensitivity, or relieving the overactive reaction without causing the nerve injury. The electrical stimulation device has an effect of regulating nerves by regulating protein and gene expression of the protein.

### BRIEF DESCRIPTION OF THE DRAWINGS

FIG. 1 is a functional block diagram of the electrical stimulation device according to some embodiments;
FIG. 2 is a schematic diagram of an example of the electrical stimulation device of FIG. 1;
FIG. 3 is a use schematic diagram of an example of the electrical stimulation device of FIG. 1;
FIG. 4 is a use schematic diagram of one other example of the electrical stimulation device of FIG. 1;
FIG. 5 is a use schematic diagram of another example of the electrical stimulation device of FIG. 1;
FIG. 6A is a schematic diagram of an example of the electrical stimulation signal generated by the electrical stimulator of FIG. 1;
FIG. 6B is a schematic diagram of one other example of the electrical stimulation signal generated by the electrical stimulator of FIG. 1;
FIG. 6C is a schematic diagram of another example of the electrical stimulation signal generated by the electrical stimulator of FIG. 1;
FIG. 7 is a line graph showing the test results of rat hind paw withdrawal thresholds;
FIG. 8A is a histogram of the test results of the pixel density of the MBP content;
FIG. 8B is a histogram of the test results of the pixel density of the NF200 content;
FIG. 8C is a histogram of the test results of the percentage of positive cells of MBP and NF200;
FIG. 9 is a graph showing the test results of fluorescent staining of MBP and NF200;
FIG. 10 is a histogram of the test results of the percentage of positive neurons of BDNF;
FIG. 11 is a graph showing the test results of fluorescent staining of BDNF;
FIG. 12 is a histogram of the test results of the percentage of positive neurons of COX-2;
FIG. 13 is a histogram of the test results of the percentage of positive neurons of c-Myc;
FIG. 14 is a histogram of the test results of the percentage of positive neurons of SP;
FIG. 15 is a histogram of the test results of the percentage of positive neurons of MEK; and
FIG. 16 is a histogram of the test results of the percentage of positive neurons of c-Fos.

### DETAILED DESCRIPTION

The term "electrical stimulation signal" used herein refers to an electrical signal transmitted by an electrical stimulator through an electrode assembly to an organism. For example, the electrical stimulation signal may be quantified into voltage, current, electric field, power, joule energy or other electrical measurement values.

The term "target region" refers to a neural tissue and/or non-neural tissue that receives the electrical stimulation signal. The neural tissue includes neurons. The non-neural tissue includes neurogliocytes, myelin sheaths, immune cells, connective tissues, epithelial cells, cardiovascular cells and/or blood cells and the like. The neurogliocytes include astrocytes, oligodendrocytes, ependymal cells, radial glial cells, Schwann cells, satellite cells, microglial cells and/or pituitary cells and the like.

The term "organism" refers to an individual, which may be a human or an animal, receiving the electrical stimulation signal, but is not limited thereto.

It should be understood that, unless explicitly defined otherwise, the terms "a" and "an" herein are not intended to limit only one, and may, under a reasonable range, refer to a single one, any one of a plurality or one kind.

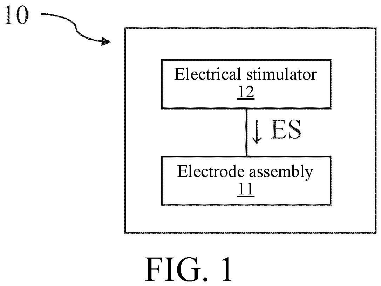

Refer to FIGs. 1-3. An electrical stimulation device 10 includes an electrode assembly 11 and an electrical stimulator 12. The electrical stimulator 12 is coupled to the electrode assembly 11 and generates an electrical stimulation signal ES. The electrode assembly 11 receives the electrical stimulation signal ES and transmits it to a target region T of an organism 20. In other words, the electrical stimulation signal ES is transmitted to the target region T through the electrode assembly 11. In some embodiments, the electrical stimulation device 10 may be of transcutaneous, transcranial, partially invasive, minimally invasive or implantable. In some embodiments, the target region T is where to be electrically stimulated by the electrical stimulation device 10, such as a central nervous system and its tissue or a peripheral nervous system and its tissue. The central nervous system and its tissue contain brain, brain stem and spinal cord; and the peripheral nervous system and its tissue contain nerves, nerve roots and root ganglia other than the brain, the brain stem and the spinal cord. In some embodiments, the peripheral nervous system and its tissue are functionally classified as an autonomic nervous system and a somatic nervous system, containing, for example, cranial nerves, vagus nerves, occipital nerves, trigeminal nerves, hypoglossal nerves, sphenopalatine ganglia, sacral nerves, lumbar nerves, pudendal nerves, sacral nerves, coccygeal nerves and the like.

In some embodiments, the electrical stimulation device 10 may be a spinal cord electrical stimulation (SCS) device, a transcutaneous electrical nerve stimulation (TENS) device, a peripheral nerve electrical stimulation (PNS) device, a deep brain electrical stimulation (DBS) device or a vagus nerve electrical stimulation (VNS) device and the like.

In some embodiments, the electrode assembly 11 and the electrical stimulator 12 may be separable elements connected to each other or integrally formed. The separable electrode assembly 11 and electrical stimulator 12 are directly or indirectly coupled (e.g., clamped, snap-fit, pluggable or electrically coupled).

In some embodiments, one or more electrical stimulation devices 10 may be positioned on the target region T. For example, a user may use multiple electrical stimulation devices 10 simultaneously to apply an electrical stimulation to the same target region T. Of course, a plurality of the electrical stimulation devices 10 may be positioned on the same organism 20 to apply an electrical stimulation to different target regions T.

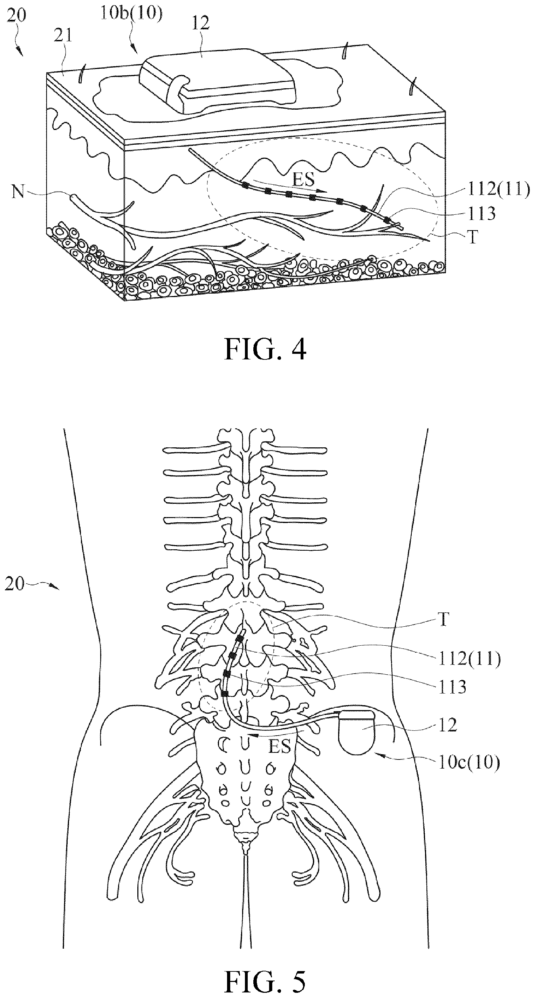

In some embodiments, the electrode assembly 11 contains an electrode of paddle-shaped, cuff-shaped, spiral, wire-shaped, thin-probe-shaped, cylindrical or the like; and the electrode assembly 11 may be linear, spiral, patch-shaped, cable-shaped, needle-shaped or the like. Preferably, the electrode of the electrode assembly 11 is close to a target nerve N of the target region T (see FIG. 4). Besides, the distance between the electrode of the electrode assembly 11 and the target nerve N is within 2 cm, even within 1.5 cm or within 1 cm.

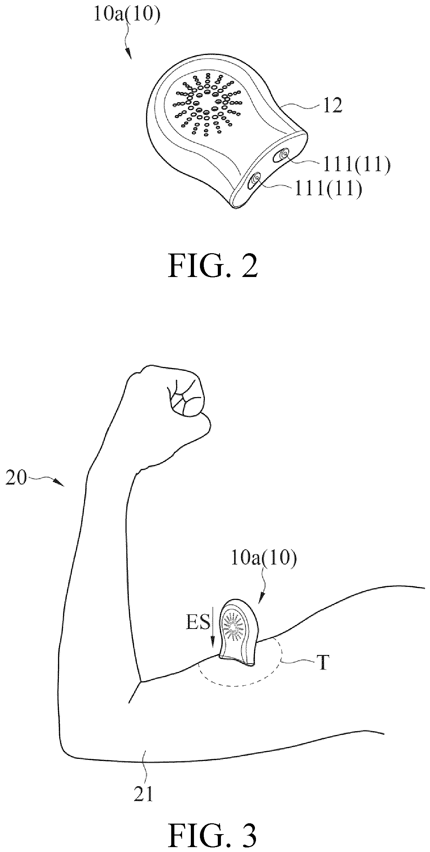

Refer to FIGs. 2-3. In some embodiments, the electrical stimulation device 10 may be a transcutaneous electrical stimulation device 10a, and the electrode assembly 11 is embedded in a surface of the electrical stimulator 12. In use, the electrical stimulator 12 is positioned on a skin 21 of the organism 20 and the surface having the electrode assembly 11 is attached to the skin 21. In other words, both the electrical stimulator 12 and the electrode assembly 11 of the electrical stimulation device 10a are positioned outside a body of the organism. The electrical stimulation signal ES generated by the electrical stimulator 12 stimulates the skin 21 and superficial nerves under the skin 21 via the electrode assembly 11. In this embodiment, the target region T contains the skin 21 and the superficial nerves under the skin 21. The skin 21 may be the surface of head, upper limbs, lower limbs, trunk, genitals or the like, and the superficial nerves under the skin 21 may be nerves within about 1, 2, 3 or 4 cm subcutaneously.

In some embodiments, the electrode assembly 11 contains dual electrodes or a plurality of electrodes such that the electrical stimulation is bipolar or tripolar. In other embodiments, the electrode assembly 11 contains a working electrode and a reference electrode such that the electrical stimulation is monopolar. As shown in FIG. 2, in a bipolar example, the electrode assembly 11 contains an electrode pair 111. In the electrical stimulation device 10a, the electrode pair 111 is positioned on the surface (i.e., a casing) of the electrical stimulator 12. In use, the electrical stimulation device 10a is attached to the skin 21 of an arm by the electrode pair 111 and is activated to output the electrical stimulation signal ES to the electrode pair 111, so as to electrically stimulate the skin 21 of the arm and the superficial nerves under the skin 21 via the electrode pair 111. In some embodiments, the superficial nerves under the skin 21 of the arm may be a musculocutaneous nerve located in biceps brachii, a radial nerve located in triceps brachii, a dorsal nerve of the penis (DPN) located in the male genitalia or a median nerve located near a wrist.

Refer to FIG. 4. In some embodiments, the electrical stimulation device 10 may be a minimally invasive electrical stimulation device 10b. After the electrode assembly 11 is implanted, one end thereof is wirelessly or wiredly connected to the electrical stimulator 12 and the other end is implanted in the organism 20. In use, the electrical stimulator 12 is positioned on the skin 21 of the organism 20 and the electrode assembly 11 is at least partially implanted under the skin 21 of the organism 20 in a minimally invasive manner. In other words, the electrical stimulator 12 of the electrical stimulation device 10b is positioned outside the body of the organism, while the electrode assembly 11 of the electrical stimulation device 10b is positioned inside the body of the organism. The electrical stimulation signal ES generated by the electrical stimulator 12 wirelessly or wiredly stimulates the target nerve N near a terminal (one end of the electrode assembly 11 not connected to the electrical stimulator 12) of the electrode assembly 11 via the electrode assembly 11. In this embodiment, the target region T is the target nerve N near the terminal of the electrode assembly 11.

As shown in FIG. 4, in an example, the electrical stimulation device 10b may be a peripheral nerve electrical stimulation device, the electrode assembly 11 may be a lead 112, and the lead 112 contains a plurality of ring electrodes 113. In the electrical stimulation device 10b, the lead 112 is wirelessly connected to the electrical stimulator 12. In use, the electrical stimulation device 10b is attached to the skin 21 of the organism 20 with the electrical stimulator 12, and the lead 112 is implanted under the skin 21 of the organism 20 in a minimally invasive manner. The electrical stimulator 12 wirelessly outputs the electrical stimulation signal ES to the lead 112, thereby electrically stimulating a nerve (i.e., the target nerve N) under the skin 21 of the organism 20 and located near the ring electrodes 113 of the lead 112 via the lead 112. In some embodiments, one end of the lead 112 may be implanted near the target nerve N and the other end may be positioned subcutaneously to enable the electrical stimulator 12 to be wirelessly connected and powered (as shown in FIG. 4). In other embodiments, the other end of the lead 112 may extend over the surface of the skin 21 to connect to the electrical stimulator 12 and be powered by a wired connection.

In some embodiments, the target region T is a nerve or neural structure around the ring electrodes 113 on the lead 112. For example, the target region T is the target nerve N around the ring electrodes 113 on the lead 112.

FIG. 5 is a use schematic diagram of another example of the electrical stimulation device of FIG. 1. Refer to FIG. 5, the electrical stimulation device 10 is an implantable electrical stimulation device 10c, such as a spinal cord electrical stimulation device or a sacral nerve electrical stimulation device, the electrode assembly 11 may be a lead 112, and the electrode assembly 11 is connected to the electrical stimulator 12. In use, both the electrical stimulator 12 and electrode assembly 11 are implanted in the organism 20, the ring electrodes 113 of the electrode assembly 11 are positioned at the terminal of the lead 112 (one end of the electrode assembly 11 not connected to the electrical stimulator 12), and the ring electrodes 113 corresponds to the position of the target region T of the organism 20. The electrical stimulation signal ES generated by the electrical stimulator 12 stimulates the nerve near the ring electrodes 113 via the ring electrodes 113 on the electrode assembly 11. In other words, the electrical stimulator 12 can be implanted at any position around the target region T of the organism 20 according to the placement position and length of the ring electrodes 113 of the lead 112. In use of the electrical stimulation device 10c, the electrical stimulator 12 is connected to one end of the lead 112 and implanted in the epidural space of the spine or near the sacral nerve around the buttocks of the organism 20. Therefore, when the electrical stimulator 12 outputs the electrical stimulation signal ES to the lead 112, the electrical stimulation signal ES can electrically stimulate the spinal cord or the sacral nerve around the buttocks of the organism 20 via the lead 112.

In some embodiments, the electrical stimulation signal ES may be a biphasic electrical stimulation signal ES. In other embodiments, the electrical stimulation signal ES may be a monophasic electrical stimulation signal ES.

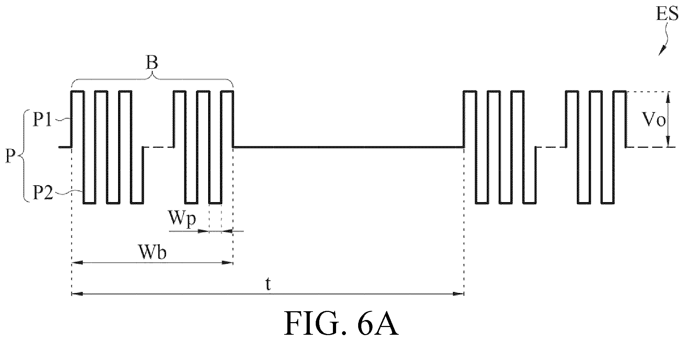

FIG. 6A is a schematic diagram of an example of the electrical stimulation signal generated by the electrical stimulator of FIG. 1. Refer to FIG. 6A. In some embodiments, the waveform of the electrical stimulation signal ES generated by the electrical stimulation device 10 in the period time t is for example, biphasic, square-wave, symmetric and charge-balanced. Of course, the waveform of the electrical stimulation signal ES may be a monophasic signal, a triangular wave, a sine wave, an asymmetric signal, or a charge-unbalanced signal or the like.

In some embodiments, the electrical stimulation signal ES contains a plurality of burst signals B, and the burst signals B have the burst frequency between 0.1 Hz and 1,000 Hz. Each burst signal B contains a plurality of pulses P, and the pulses P have the pulse frequency between 1,000 Hz and 1,000 kHz. Preferably, the burst signals B have the burst frequency between 0.1 Hz and 500 Hz, for example, 0.1 Hz-400 Hz, 0.1 Hz-200 Hz, 0.1 Hz-100 Hz or 0.1 Hz-20 Hz. Preferably, the pulses P have the pulse frequency between 1 kHz and 750 kHz, for example, 400 kHz-600 kHz.

The wave width Wb of the burst signals B is between 1 ms and 10 s, and the pulse width Wp of the pulses P is between 0.5 µs and 1 ms. Preferably, the wave width Wb of the burst signals B is between 2 ms and 500 ms, for example, between 10 ms and 50 ms. Preferably, the pulse width Wp of the pulses P is between 1 µs and 1 ms, for example, between 1 µs and 100 µs.

In some embodiments, the current intensity of the electrical stimulation signal ES may be between 0.1 mA and 120 mA, and the voltage intensity Vo of the electrical stimulation signal ES may be between 0.05 V and 60 V Preferably, the current intensity is between 6 mA and 40 mA. Preferably, the voltage intensity Vo is between 3 V and 20 V

In some embodiments, the pulses P are biphasic and successive of opposite polarity. The pulses P contained in the biphasic pulses include positive pulses P1 and negative pulses P2, the positive pulses P1 and the negative pulses P2 alternately appear, and preferably, the positive pulses P1 and the negative pulses P2 are charge balanced. In some embodiments, the pulses P may be a square wave, a sine wave, a triangular wave or a combination thereof. In some embodiments, the burst signals B may be a square wave, a sine wave, a triangle wave, a symmetric wave, an asymmetric wave or a combination thereof.

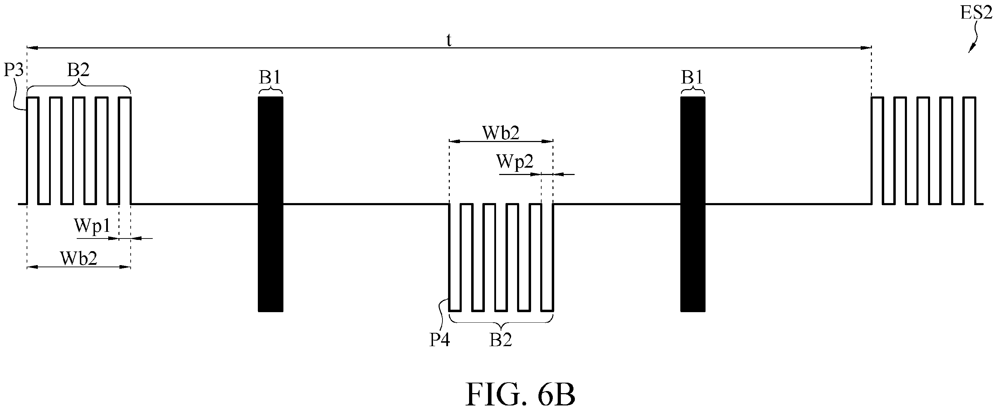

FIG. 6B is a schematic diagram of one other example of the electrical stimulation signal generated by the electrical stimulator of FIG. 1. Refer to FIG. 6B. In some embodiments, the electrical stimulation signal ES generated by the electrical stimulation device 10 is an electrical stimulation signal ES2, the electrical stimulation signal ES2 is exemplified by containing two alternate burst signals B1 and B2, wherein the burst signals B1 are the same as the burst signals B in FIG. 6A, and the waveform thereof is illustrated by a solid block, which is not described herein again. The burst frequency of the burst signals B2 is also between 0.1 Hz and 1,000 Hz, and preferably, the burst frequency of the burst signals B2 is less than or equal to that of the burst signals B1. Here, the burst signals B1 and B2 alternately appear at 1:1 in time.

In some embodiments, the burst signals B2 contain a plurality of positive pulses P3 or a plurality of negative pulses P4. For example, referring to FIG. 6B, the electrical stimulation signal ES2 contains two burst signals B2, first burst signals B2 contain a plurality of positive pulses P3 and second burst signals B2 contain a plurality of negative pulses P4, the two burst signals B2 have the same waveform but opposite phases. The pulse frequency of the positive pulses P3 and the negative pulses P4 is in the range of 1 kHz to 1,000 kHz, and preferably the pulse frequency of the positive pulses P3 and the negative pulses P4 is in the range of 1 kHz to 300 kHz. Preferably, the pulse frequency of the positive pulse P3 or the negative pulse P4 contained in the burst signals B2 is smaller than that of the pulses P contained in the burst signals B1. For example, the pulse frequency of the positive pulse P3 or the negative pulse P4 contained in the burst signals B2 is smaller than 1/3 of that of the pulses P contained in the burst signals B1.

In some embodiments, the wave width Wb2 of the burst signals B2 is also between 1 ms and 10 s, and preferably, the wave width Wb2 of the burst signals B2 is between 2 ms and 500 ms, for example, between 5 ms and 100 ms.

In some embodiments, the pulse widths Wp1 and Wp2 of the positive pulses P3 and the negative pulses P4 are also between 1 µs and 1 ms, and preferably, the pulse widths Wp1 and Wp2 of the positive pulses P3 and the negative pulses P4 are between 3.3 µs and 1 ms, for example, 1 µs. The pulse frequencies and the pulse widths Wp1 and Wp2 of the positive pulses P3 and the negative pulses P4 may be the same or different. Taking the same pulse frequencies and pulse widths as an example herein, the charge of the positive pulses P3 is equal to that of the negative pulses P4 in a unit time, thereby achieving a delayed charge balance and reducing erosion of an electrode due to polarization.

In some embodiments, when the accumulated charge of the positive pulses P3 or the negative pulses P4 of the burst signals B2 is greater than or equal to 20 µC (microcoulomb), it can be sensible by the organism 20 (a user using the electrical stimulation device 10), such as a tapping sensation, a shaking sensation or a tingling sensation, such that the organism 20 can know that the electrical stimulation is in progress.

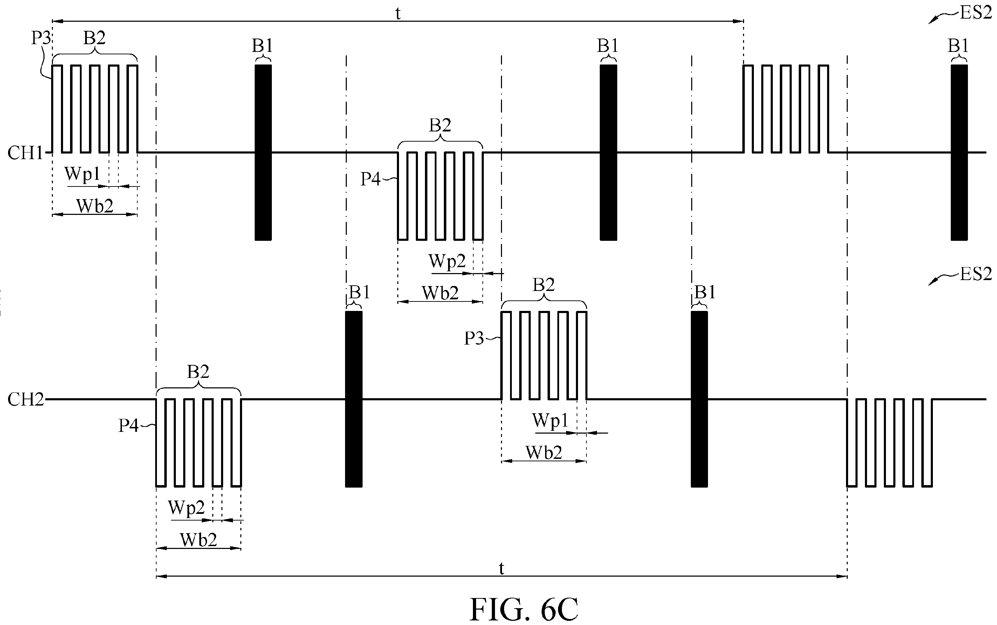

FIG. 6C is a schematic diagram of another example of the electrical stimulation signal generated by the electrical stimulator of FIG. 1. Refer to FIG. 6C. In some embodiments, the electrical stimulation device 10 has two output ends CH1 and CH2. In this state, the two output ends CH1 and CH2 respectively output the electrical stimulation signals ES2, and the output times of the electrical stimulation signals ES2 at the two output ends CH1 and CH2 are not overlapped in each burst signal or each pulse on period in the period time t. For example, the on periods of the burst signals B1 and burst signals B2 at the output end CH1 do not overlap with those of the burst signals B1 and burst signals B2 at the output end CH2. In this embodiment, the voltage or current interference of the two output ends CH1 and CH2 when outputting the electrical stimulation signal ES2 can be reduced, thereby avoiding the instability of the outputs of the two output ends CH1 and CH2. For example, referring to FIG. 5, the electrical stimulation device 10c may contain two leads 112 (FIG. 5 only shows one lead 112 and the other lead 112 is not shown), which respectively correspond to the two output ends CH1 and CH2 to output the electrical stimulation signal ES2 to the plurality of ring electrodes 113 on the two leads 112 for bipolar output of the electrical stimulation signal ES2.

In some embodiments, the electrical stimulation signal ES generated by the electrical stimulation device 10 may reduce pain related neuropeptide, neuroinflammatory information and/or neuropathic pain signals of the organism 20.

In some embodiments, the electrical stimulation signal ES generated by the electrical stimulation device 10 reduces the level of COX-2 of the organism 20. In some embodiments, the electrical stimulation signal ES generated by the electrical stimulation device 10 inhibits the level of the COX-2 of the organism 20. In some embodiments, the electrical stimulation signal ES generated by the electrical stimulation device 10 reduces the level of the COX-2 by inhibiting the COX-2 protein or the gene expression of the COX-2 protein. In some embodiments, reducing the level of the COX-2 of the organism 20 in the body occurs within 24 hours after receiving the electrical stimulation signal ES at the target region T. In some embodiments, the electrical stimulator 12 generates the electrical stimulation signal ES for a cumulative time less than or equal to 12 hours per day so as to reduce the level of the COX-2 of the organism 20.

In some embodiments, the electrical stimulation signal ES generated by the electrical stimulation device 10 reduces the levels of brain-derived neurotrophic factor (BDNF) and/or substance P of the organism 20.

In some embodiments, the electrical stimulator 12 uses the electrical stimulation signal ES to inhibit the neuroinflammatory response of the organism 20 via the electrode assembly 11.

In some embodiments, the electrical stimulator 12 uses the electrical stimulation signal ES to inhibit and/or relieve pain of the organism 20. In some embodiments, the electrical stimulator 12 uses the electrical stimulation signal ES to inhibit and/or relieve the generation of the neuropathic pain signals of the organism 20 via the electrode assembly 11. In some embodiments, the electrical stimulation signal ES generated by the electrical stimulation device 10 inhibits and/or relieves at least a part of pain on a nerve conduction path of the target region T. In some embodiments, the pain of the organism 20 may be acute or chronic.

In some embodiments, the electrical stimulation device 10 generates the electrical stimulation signal ES for a duration time of at least 3 minutes to 60 minutes. In some embodiments, the electrical stimulation device 10 generates the electrical stimulation signal ES for a duration time of 5 minutes to 60 minutes, 5 minutes to 30 minutes, or 5 minutes to 20 minutes. Preferably, the electrical stimulation device 10 generates the electrical stimulation signal ES for a duration time of 15 minutes.

In some embodiments, the electrical stimulation device 10 generates the electrical stimulation signal ES with a cumulative time per day of less than or equal to 12 hours, preferably less than 10 hours, 8 hours, 6 hours, 4 hours, 2 hours, or 1 hour so as to relieve pain, relieve symptoms, desensitize overactive nerves, or relieve overactive reaction. In other words, after the organism 20 receives the electrical stimulation signal ES for less than or equal to 12 hours, the electrical stimulation signal ES relieves the pain of the organism 20, relieves the symptoms of the organism 20, reduces the overactive neural sensitivity of the organism 20 or relieves the overactive reaction of the organism 20.

In some embodiments, the electrical stimulator 12 uses the electrical stimulation signal ES to inhibit and/or relieve the pain of the organism 20 sustaining for a particular time via the electrode assembly 11. In some embodiments, the particular time is at least one hour, one hour to 4 days, one hour to 5 days; at least 1 day, 1 day to 4 days, or 1 day to 5 days.

In some embodiments, the target region T is a dorsal root ganglion.

In some embodiments, the electrical stimulation signal ES generated by the electrical stimulation device 10 has effects of inhibiting and/or relieving acute pain, chronic pain, local pain, spasticity, renal hypertension, overactive bladder, palmar hyperhidrosis, premature ejaculation, or diseases or symptoms caused by nerve sensitization.

### Test I: Animal test-Mechanical allodynia evaluation test

### A. Test process:

### (1) Rat CCI model establishment

The sciatic nerves of the left hind paws of normal rats (Sprague Dawley, SD) were ligated with 5-0 nylon wires so as to establish a model of chronic constriction injury (CCI) through controllable forces (6 g rope tension) monitored by a computer. Compressive neuropathy of the CCI model had marked and sustained mechanical allodynia and increased neuroinflammation. The CCI model treated with the nerve injury was a CCI group, which was divided into a CCI-UHF group and a CCI+UHF group with 6 rats in each group. The CCI model can be established according to the reference of Chen et al (Chen et al, 2020).

### (2) First ultrahigh frequency electrical stimulation treatment T1

After the CCI model was established for 7 days, an electrical stimulation device (StimOn GM2439; Gimer Medical Co., Ltd., Taiwan) was placed on the skin above the sciatic nerves of the rats in the CCI+UHF group and the rats were continuously treated with an ultrahigh frequency electrical stimulation signal for 15 minutes. An electrode pad of the electrical stimulation device was firmly attached to the skin above the sciatic nerves (lesions). The ultrahigh frequency electrical stimulation signal contained a plurality of burst signals, and the burst signals contained a plurality of pulses. The burst frequency of the burst signals was 2 Hz, the pulse frequency of the pulses was 500 kHz, and the voltage intensity of the electrical stimulation signal was about ±6.6 V The duration time of the electrical stimulation signal was 15 minutes. The rats in the CCI-UHF group was not subjected to the first ultrahigh frequency electrical stimulation treatment T1.

### (3) Second ultrahigh frequency electrical stimulation treatment T2

4 days after the first ultrahigh frequency electrical stimulation treatment T1, the rats in the CCI+UHF group were subjected to a second ultrahigh frequency electrical stimulation treatment T2. The treatment process was the same as that of the first ultrahigh frequency electrical stimulation treatment T1 in (2), which was not described herein again. The rats in the CCI-UHF group was not subjected to the second ultrahigh frequency electrical stimulation treatment T2.

### (4) Mechanical allodynia assessment test

A monofilament tactile pain test (von Frey test) was used to measure the threshold forces (withdrawal thresholds) required to cause the withdrawal of the paws of the rats in the CCI-UHF group and CCI+UHF group to obtain the plantar tolerance of the rats, thereby evaluating the mechanical allodynia of the normal rats. The underside of the rat hind paws was poked with a Semmes-Weinstein monofilament (SWM; North Coast Medical Inc., USA). During the test, each rat was subjected to two monofilament tactile pain tests, and the interval time of the two monofilament tactile pain tests was at least 10-15 minutes.

Before the CCI model was established, 7 days after the CCI model was established, but the electrical stimulation was not performed, 30 minutes, 1 day, 2 days, 3 days, 4 days and 5 days after the first ultrahigh frequency electrical stimulation treatment T1, and 30 minutes, 1 day, 2 days, 3 days, 4 days and 5 days after the second ultrahigh frequency electrical stimulation treatment T2, the rats in the CCI-UHF group and CCI+UHF group were respectively subjected to the mechanical allodynia assessment test.

The withdrawal threshold measured before the CCI model was established was the withdrawal threshold at day -7. The withdrawal threshold measured 7 days after the CCI model was established, but the electrical stimulation was not performed was the withdrawal threshold at day 0. The withdrawal thresholds measured after the first ultrahigh frequency electrical stimulation treatment T1 were the withdrawal thresholds at 30 minutes, 1 day, 2 days, 3 days, 4 days, and 5 days after the first ultrahigh frequency electrical stimulation treatment T1. The withdrawal thresholds measured after the second ultrahigh frequency electrical stimulation treatment T2 were the withdrawal thresholds at 30 minutes, 1 day, 2 days, 3 days, 4 days, and 5 days after the second ultrahigh frequency electrical stimulation treatment T2.

### (5) Statistical analysis

The withdrawal thresholds between groups were compared by a Student's t-test and analyzed. The withdrawal thresholds were all expressed as mean standard deviation. **** indicated that compared to the CCI-UHF group, the CCI+UHF group was statistically significant, p < 0.0001; and ++++ indicated that compared to the CCI+UHF group at day 0, the CCI+UHF group at different time points was statistically significant, p < 0.0001.

### B. Test results:

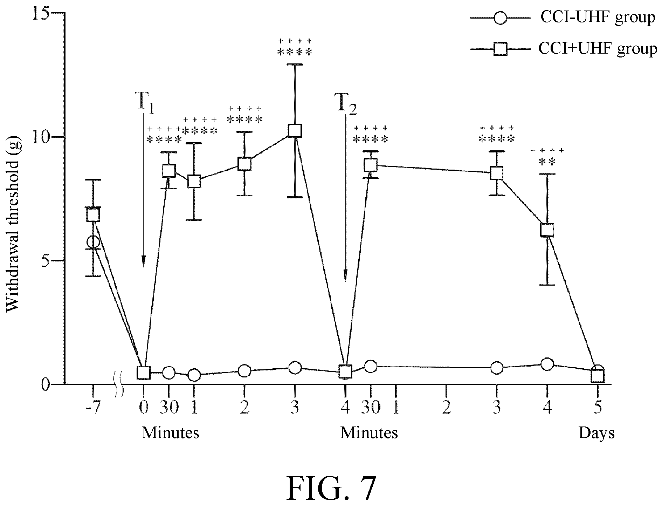

Refer to FIG. 7. On day -7, before the CCI model was established, the withdrawal threshold of the CCI-UHF group was 5.77±1.39 g, while the withdrawal threshold of the CCI+UHF group was 6.86±1.40 g. On day 0, namely 7 days after the CCI model was established, but the electrical stimulation was not performed, the withdrawal threshold of the CCI-UHF group was 0.45±0.068 g, while the withdrawal threshold of the CCI+UHF group was 0.48±0.087 g. At 30 minutes after the first ultrahigh frequency electrical stimulation treatment T1, the withdrawal threshold of the CCI+UHF group was 8.64±0.73 g. At 3 days after the first ultrahigh frequency electrical stimulation treatment T1, the withdrawal threshold of the CCI+UHF group was 10.25±2.68 g. At 4 days after the first ultrahigh frequency electrical stimulation treatment T1 (second ultrahigh frequency electrical stimulation treatment T2 was not performed), the withdrawal threshold of the CCI+UHF group was 0.55±0.20 g. Then the second ultrahigh frequency electrical stimulation treatment T2 was performed 4 days after the first ultrahigh frequency electrical stimulation treatment T1. 30 minutes after the second ultrahigh frequency electrical stimulation treatment T2, the withdrawal threshold of the CCI+UHF group was 8.53±0.89 g. At 5 days after the second ultrahigh frequency electrical stimulation treatment T2, the withdrawal threshold of the CCI+UHF group was 0.34±0.17 g. On the other hand, the withdrawal threshold of the CCI-UHF group was consistently maintained at about 0.45±0.068 g for 2 weeks.

Therefore, the ultrahigh frequency electrical stimulation treatment can relieve the mechanical allodynia caused by the nerve injury treatment. Besides, the first ultrahigh frequency electrical stimulation treatment T1 may increase the decreased withdrawal threshold continuously for 3 days, while the second ultrahigh frequency electrical stimulation treatment T2 may increase the decreased withdrawal threshold continuously for 4 days. The ultrahigh frequency electrical stimulation treatment inhibits and/or relieves the mechanical allodynia for at least about 4 days. The electrical stimulation signal generated by the electrical stimulator has the effect of relieving and/or inhibiting neuropathic pain caused by the nerve injury treatment.

### Test II: Animal test-neuronal injury test

### A. Test process:

### (1) First ultrahigh frequency electrical stimulation treatment T1

Normal rats (Sprague Dawley, SD) without injury treatment were a control group (Ctrl group) which was divided into a Ctrl-UHF group (4 rats) and a Ctrl+UHF group (3 rats). The electrical stimulation device (StimOn GM2439; Gimer Medical Co., Ltd., Taiwan) was placed on the skin above the sciatic nerves of the rats in the Ctrl+UHF group and the rats were continuously treated with an ultrahigh frequency electrical stimulation signal for 15 minutes. An electrode pad of the electrical stimulation device was firmly attached to the skin above the sciatic nerves. The ultrahigh frequency electrical stimulation signal contained a plurality of burst signals, and the burst signals contained a plurality of pulses. The burst frequency of the burst signals was 2 Hz, the pulse frequency of the pulses was 500 kHz, the voltage intensity of the electrical stimulation signal was about ±6.6 V, and the duration time of the electrical stimulation signal was 15 minutes. The pulses were a symmetric biphasic sine wave. In addition, the rats in the Ctrl-UHF group was not subjected to the first ultrahigh frequency electrical stimulation treatment T1.

### (2) Second ultrahigh frequency electrical stimulation treatment T2

4 days after the first ultrahigh frequency electrical stimulation treatment T1, the rats in the Ctrl+UHF group were subjected to a second ultrahigh frequency electrical stimulation treatment T2. The treatment process was the same as that of the first ultrahigh frequency electrical stimulation treatment T1 in (1), which was not described herein again. The rats in the Ctrl-UHF group was not subjected to the second ultrahigh frequency electrical stimulation treatment T2.

### (3) Immunofluorescent staining assay

14 days after the first ultrahigh frequency electrical stimulation treatment T1, the middle sciatic nerves of the left hind paws of the rats in the Ctrl+UHF group and the Ctrl-UHF group were taken out for immunofluorescent staining assay of myelin basic protein (MBP) and neurofilament-200 (NF200).

The immunofluorescent staining assay was performed according to the general immunofluorescent (IF) staining test process. Staining reagents contained a mouse anti-neurofilament heavy polypeptide antibody (mouse anti-NF200, 1: 200; product number N0142, Sigma-Aldrich, USA) and a rabbit anti-MBP antibody (1:200; product number GTX133108, GeneTex, USA) as primary antibodies, and a goat anti-mouse IgG antibody (1:200; GeneTex, product number GTX213111-05) as a secondary antibody of NF200.

### (4) Quantification of staining results

After the staining was completed, images were observed and photographed by using a fluorescence microscope (model BX61, purchased from Olympus, Japan), and analyzed by using an ImageJ software (version 1.53s, NIH, USA) so as to further obtain the levels of proteins of MBP and NF200. The ImageJ software calculated a fluorescence signal per pixel. The MBP was used as a staining marker for Schwann cells and the NF200 as a staining marker for mature axons.

### (5) Statistical analysis

The levels of the MBP and the NF200 and the fluorescent staining results of the MBP and the NF200 among groups were compared by a Student's t-test and analyzed. The levels of the MBP and the NF200 and the fluorescent staining results of the MBP and the NF200 were expressed as mean standard deviation (SD). ns indicated that there was no significant difference between the Ctrl+UHF and Ctrl-UHF groups.

### B. Test results:

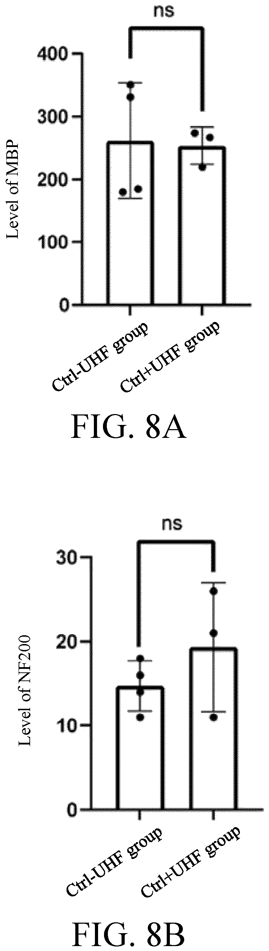

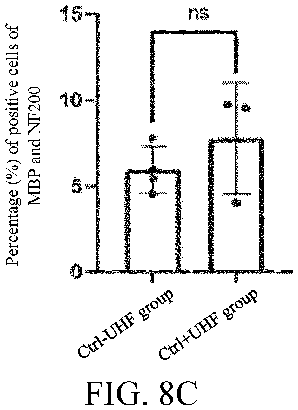

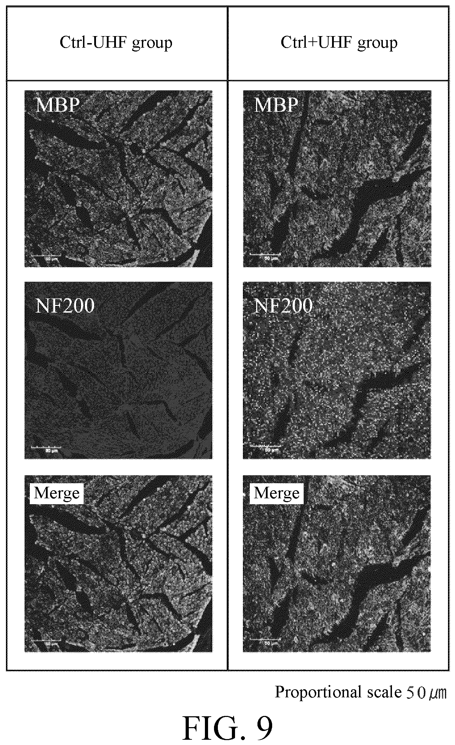

Refer to FIGs. 8A, 8B, 8C and 9, there were no significant differences in the levels of the MBP and the NF200 and the fluorescent staining results of the MBP and the NF200 in the Ctrl+UHF and Ctrl-UHF groups.

Therefore, the number of myelinated axons of the uninjured nerves of the rats treated with the ultrahigh frequency electrical stimulation treatment was not significantly different from that of the uninjured nerves of the rats not treated with the ultrahigh frequency electrical stimulation treatment. The ultrahigh frequency electrical stimulation treatment did not injure the axons and Schwann cells in the uninjured nerves.

### Testing III: Animal test-nerve regulation test

### A. Test process:

### (1) Rat CCI model establishment

The sciatic nerves of the left hind paws of normal rats (Sprague Dawley, SD) were ligated with 5-0 nylon wires so as to establish a model of chronic constriction injury (CCI) through controllable forces (6 g rope tension) monitored by a computer. Compressive neuropathy of the CCI model had marked and sustained mechanical allodynia and increased neuroinflammation. The CCI model treated with the nerve injury was a CCI group and the normal rats not treated with the injury was a control group (Ctrl group). The CCI group was divided into a CCI-UHF group and a CCI+UHF group. The Ctrl group was a Ctrl-UHF group. The CCI model can be established according to the reference of Chen et al (Chen et al, 2020).

### (2) Ultrahigh frequency electrical stimulation treatment

After the CCI model was established for 7 days, an electrical stimulation device (StimOn GM2439; Gimer Medical Co., Ltd., Taiwan) was placed on the skin above the sciatic nerves of the rats in the CCI+UHF group and the rats were continuously treated with an ultrahigh frequency electrical stimulation signal for 15 minutes. An electrode pad of the electrical stimulation device was firmly attached to the skin above the sciatic nerves (lesions). The ultrahigh frequency electrical stimulation signal contained a plurality of burst signals, and the burst signals contained a plurality of pulses. The burst frequency of the burst signals was 2 Hz, the pulse frequency of the pulses was 500 kHz, and the voltage intensity of the electrical stimulation signal was about ±6.6 V The duration time of the electrical stimulation signal was 15 minutes.

The rats in the CCI-UHF group and the Ctrl-UHF group were not subjected to the ultrahigh frequency electrical stimulation treatment.

### (3) Immunofluorescent staining assay

On the day after the CCI model was established, left L4-L5 dorsal root ganglia (DRG) of the rats in the Ctrl-UHF group (not subjected to the nerve injury and not subjected to the electrical stimulation treatment) were taken out for immunofluorescent staining assay.

At 7 days after the CCI model was established, left L4-L5 DRG of the rats in the CCI-UHF group (subjected to the nerve injury, but not subjected to the electrical stimulation treatment) were taken out for immunofluorescent staining assay.

At 1 day after the ultrahigh frequency electrical stimulation treatment, left L4-L5 DRG of the rats in the CCI+UHF group were taken out for immunofluorescent staining assay, which was recorded as CCI+UHF 1-day group. At 5 days after the ultrahigh frequency electrical stimulation treatment, the left L4-L5 DRG of the rats in the CCI+UHF group were taken out for immunofluorescent staining assay, which was recorded as CCI+UHF 5-day group.

The immunofluorescent staining assay was performed according to the general immunofluorescent (IF) staining test process. The staining reagents contained a rabbit anti-c-FOS antibody (purchased from GeneTex), a rabbit anti-brain-derived neurotrophic factor (BDNF) antibody (purchased from Elabscience), a rabbit anti-COX-2 antibody (purchased from Elabscience), a rabbit anti-c-Myc antibody (purchased from GeneTex), a guinea pig anti-substance P (SP) antibody (purchased from GeneTex) and a rabbit anti-MEK antibody (purchased from GeneTex) as primary antibodies, and a goat anti-guinea pig IgG antibody (purchased from GeneTex) as a secondary antibody of SP and a goat anti-rabbit IgG antibody (purchased from GeneTex) as secondary antibodies of BDNF, COX-2, c-Myc, MEK and c-FOS.

After the staining was completed, images were observed and photographed by using a fluorescence microscope (model BX61, purchased from Olympus, Japan), and analyzed by using an ImageJ software (version 1.53s, NIH, USA) so as to obtain the content ratio of pain related neuropeptides BDNF, c-Myc, and COX-2, and neuroinflammatory information SP, MEK (mitogen-activated protein kinase (MAPK) kinase) and c-Fos. Neurons with stronger fluorescence signals of the C-Fos, BDNF, COX-2, c-Myc, SP and MEK, indicating that the neurons were marked to be positive by the antibodies corresponding to the proteins. The higher ratios of the positive neurons of the c-Fos, BDNF, COX-2, c-Myc, SP and MEK in the total neurons indicated the higher levels of the c-Fos, BDNF, COX-2, c-Myc, SP and MEK.

### (5) Statistical analysis

The experimental data between groups were compared with a Student's t-test and analyzed. The experimental data were all expressed as mean standard deviation (SD). * indicated p < 0.05, ** indicated p < 0.01, *** indicated p < 0.001, **** indicated p < 0.00001, and ns indicated no significant difference.

### B. Test results:

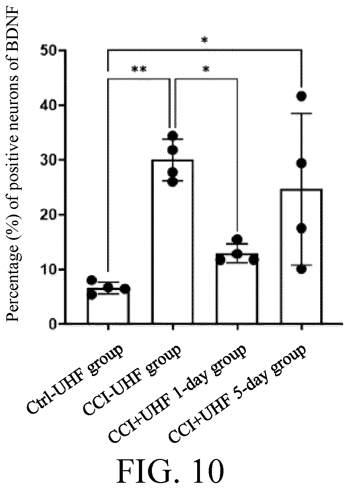

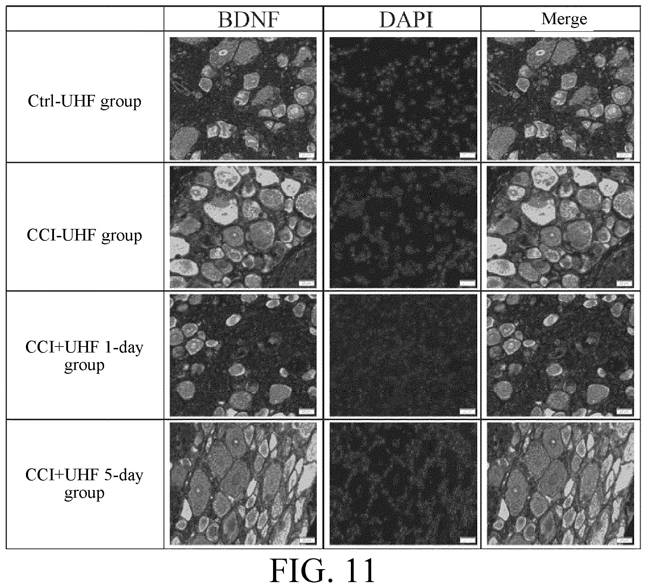

Refer to FIGs. 10 and 11, the percentage of positive neurons of the BDNF in the CCI-UHF group was 29.98%±3.79%, the percentage of positive neurons of the BDNF in the CCI+UHF 1-day group was 12.95%±1.71%, and the percentage of positive neurons of the BDNF in the CCI+UHF 5-day group was 24.66%±13.85%.

That is, the level of the BDNF was significantly increased by the nerve injury treatment, but significantly decreased in the CCI+UHF 1-day group after the ultrahigh frequency electrical stimulation treatment. The level of the BDNF in the CCI+UHF 5-day group returned to that in the CCI-UHF group (subjected to the nerve injury, but not subjected to the electrical stimulation treatment), and had no significant difference with that in the CCI-UHF group.

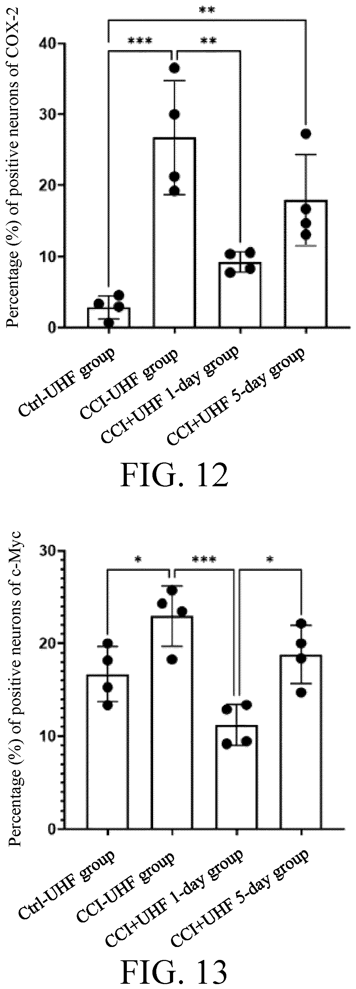

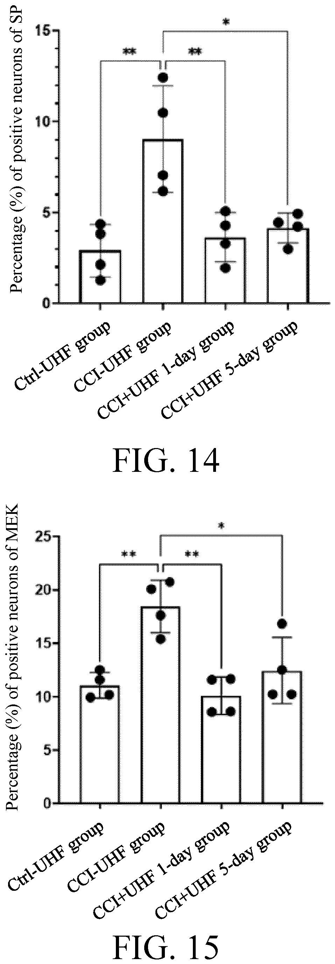

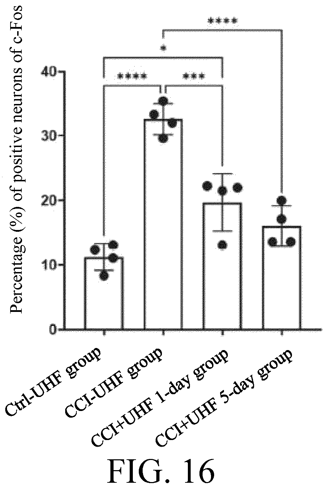

Refer to FIGs. 12 and 13, c-Myc and COX-2 both showed the same trend except for the BDNF. The levels of the c-Myc and the COX-2 can be significantly increased by the nerve injury treatment, but the levels of the c-Myc and the COX-2 can be significantly reduced by the ultrahigh frequency electrical stimulation treatment and can be continuously reduced for at least 4 days. In addition, referring to FIGs. 14-16, substance P (SP), MEK and c-Fos also showed similar trends. After the nerve injury treatment, the levels of the substance P (SP), MEK and c-Fos were all significantly increased. However, after the ultrahigh frequency electrical stimulation treatment, the levels of the substance P (SP), MEK and c-Fos in the CCI+UHF 1-day group were significantly decreased and can be continuously reduced for at least 5 days after the ultrahigh frequency electrical stimulation treatment.

The percentages of positive neurons of the c-Myc, COX-2, substance P (SP), MEK and c-Fos in each group were shown in Table 1, which was not described herein again.

**Table 1**

| **Percentage of positive neurons** | **CCI-UHF group** | **CCI+UHF 1-day group** | **CCI+UHF 5-day group** |
|---|---|---|---|
| COX-2 | 26.75%±8.04% | 9.25%±1.44% | 17.92%±6.41% |
| c-Myc | 22.94%±3.25% | 11.22%±2.20% | 18.81%±3.14% |
| SP | 9.04%±2.92% | 3.66%±1.36% | 4.16%±0.83% |
| MEK | 18.44%±2.44% | 10.11%±1.75% | 12.45%±3.11% |
| c-Fos | 32.60%±2.41% | 19.71%±4.45% | 16.06%±3.12% |

Therefore, the ultrahigh frequency electrical stimulation treatment can reduce the levels of the pain related neuropeptides BDNF, COX-2 and c-Myc continuously for at least 4 days. The ultrahigh frequency electrical stimulation treatment can reduce the levels of the neuroinflammatory information SP, MEK and c-FOS continuously for at least 5 days.

The electrical stimulation signal generated by the electrical stimulator had the effects of reducing the pain related neuropeptides, and relieving the inflammation information and neuropathic pain.

The BDNF is critical for neuronal survival, differentiation and synaptic strength regulation and is involved in inflammatory pain mechanisms. The BDNF is considered as a neuroregulator and plays an important role in spinal plasticity. Besides, the levels of the BDNF and downstream signal proteins (such as MEK, c-Myc and c-FOS) thereof were down-regulated after the ultrahigh frequency electrical stimulation treatment. The electrical stimulation signal generated by the electrical stimulator can inhibit and/or relieve neuroinflammation and inhibit and/or relieve the neuropathic pain by regulating a BDNF/MAPK-mediated pain transduction pathway.

The level of the COX-2 is related with the development and maintenance of the neuropathic pain. Prostaglandins synthesized by the COX-2 are involved in the pathogenesis of the inflammatory response and neuropathic pain. The electrical stimulation signal generated by the electrical stimulator can inhibit and/or relieve pain or inhibit and/or relieve the inflammatory response by inhibiting the generation of the COX-2 and/or reducing the level of the COX-2. The reduction in the level of the COX-2 could mean quantitative reduction in the level of the COX-2 or mean conversion of the COX-2 into inactive state. The reduction in the level of the COX-2 is not limited to the location of the DRG, but anywhere the level of the COX-2 is detectable, such as by extraction from blood, saliva, urine, stool, spinal fluid, and other biological samples that may be sampled. A detection method may include enzyme-linked immunosorbent assay (ELISA), immunofluorescent staining, or other immunological methods.

SP can bind to its specific receptor neurokinin-1 (NK-1) which sensitizes neurons and produces sense of pain. The electrical stimulation signal generated by the electrical stimulator can inhibit and/or relieve pain by inhibiting the generation of the SP.

The test proved that the levels of the neuropeptides BDNF, c-Myc and COX-2, and neuroinflammatory information SP, MEK and c-Fos were increased due to pain or inflammation, which can be significantly decreased after the ultrahigh frequency electrical stimulation for 15 minutes. Therefore, the ultrahigh frequency electrical stimulation has a similar effect to general anti-inflammatory or analgesic drugs, becomes an electronic drug, and should be related to the regulation of BDNF/MAPK. Furthermore, the expressions of some neural information (SP, MEK and c-Fos) were inhibited for up to five days even with a single ultrahigh frequency electrical stimulation for 15 minutes. Therefore, it is possible to reduce the administration of the analgesics and further reduce the side effects of drug administration and even the risk of hemodialysis by using the electrical stimulation device of any embodiment.

In summary, according to any of the embodiments, the electrical stimulation device has the effect of reducing COX-2 without causing the nerve injury. The electrical stimulation device has the effects of reducing the level of the brain-derived neurotrophic factor, reducing the level of the substance P, inhibiting the neuroinflammatory response, relieving and/or inhibiting pain, relieving symptoms, reducing nerve sensitivity, or relieving the overactive reaction without causing the nerve injury. The electrical stimulation device has an effect of regulating nerves by regulating protein and gene expression of the protein to reduce the level of the protein.

## Claims

1. An electrical stimulation device (10), comprising
an electrode assembly (11); and
an electrical stimulator (12) coupled to the electrode assembly (11) and generates an electrical stimulation signal (ES), wherein
the electrical stimulation signal (ES) is transmitted to a target region (T) of an organism (20) via the electrode assembly (11) and contains a plurality of burst signals (B), the burst signals (B) have the burst frequency between 0.1 Hz and 1,000 Hz, each burst signal (B) contains a plurality of pulses (P), the pulses (P) have the pulse frequency between 1 kHz and 1,000 kHz, and the electrical stimulation signal (ES) reduces a level of cyclooxygenase-2 of the organism (20).

2. The electrical stimulation device (10) according to claim 1, wherein the electrical stimulation signal (ES) reduces the level of the cyclooxygenase-2 by inhibiting a protein expression of the cyclooxygenase-2.

3. The electrical stimulation device (10) according to claim 1, wherein reducing the level of the cyclooxygenase-2 of the organism (20) occurs within 24 hours after receiving the electrical stimulation signal (ES).

4. The electrical stimulation device (10) according to claim 1, wherein the electrical stimulator (10) generates the electrical stimulation signal (ES) for a cumulative time less than or equal to 12 hours per day.

5. The electrical stimulation device (10) according to claim 1, wherein the electrical stimulation signal (ES) further reduces the level of brain-derived neurotrophic factor (BDNF) and/or substance P of the organism (20).

6. The electrical stimulation device (10) according to claim 1, wherein the electrical stimulator (10) uses the electrical stimulation signal (ES) to inhibit neuroinflammatory response of the organism (20).

7. The electrical stimulation device (10) according to claim 1, wherein the electrical stimulation signal (ES) inhibits and/or relieves at least a part of pain on a nerve conduction path of the target region (T).

8. The electrical stimulation device (10) according to claim 1, wherein after the organism (20) receives the electrical stimulation signal (ES) for less than or equal to 12 hours, the electrical stimulation signal (ES) relieves pain of the organism (20), relieves symptoms of the organism (20), reduces neural sensitivity of the organism (20) or relieves overactive reaction of the organism (20) and maintains for at least 1 hour.
